# EUROPEAN PATENT SPECIFICATION

(11) **EP 3 925 635 B1**
(45) Date of publication and mention of the grant of the patent: **21.01.2026**
(21) Application number: 21167013.8
(22) Date of filing: 06.04.2021
(51) Int. Cl.: A61L 9/20, B66B 11/02

(54) **METHOD FOR SANITIZING AIR IN AN ELEVATOR CABIN WITH UV RADIATION**
VERFAHREN ZUR LUFTREINIGUNG IN EINER AUFZUGSKABINE MIT UV-BESTRAHLUNG
PROCÉDÉ DE DÉSINFECTION DE L'AIR DANS UNE CABINE D'ASCENSEUR AVEC UN RAYONNEMENT UV

(30) Priority: 19.05.2020 RU 2020116436
(43) Date of publication of application: 22.12.2021
(73) Proprietor: "LitTransServis" Limited Liability Company, 127015 Moskva (RU)
(72) Inventor: Kiknadze, Nikolai Dzhemalovich, 119019 Moskva (RU)
(74) Representative: Cabinet Chaillot

(56) References cited:
- KR-A- 20060 018 556
- RU-U1- 194 640

## Description

The invention relates to the sphere of disinfecting air in enclosed structures with a high level of air contamination. Such environments include, for example, urban vehicles, elevator cabins, etc., where it is necessary to maintain the contaminated air at an epidemiologically safe level during the short-term stay of people or animals.

Dangerous microorganisms mostly enter the air once they get emitted by a sick person. For example, when talking, a sick person can release up to 800 dangerous microorganisms per minute, and up to 40,000 microorganisms when sneezing. Given the small size of elevator cabin, the concentration of dangerous microorganisms in the cabin air environment after a sick person's ride can reach very high numbers, which poses actual danger of subsequent passengers becoming infected.

There are specific problems with air purification in enclosed structures, such as on ships, in offices, on planes and in hospitals. Up to now, air filtration has been the most widely preferred method of air sanitization, but it has proved unsatisfactory - even inefficient - in terms of energy consumption.

A well-known method of air sanitization (RU 2280473, CL. A61L9/20, F24F3/16, 2001) presupposes removing air from an enclosed space, filtering the exhaust air, passing the exhaust air over surfaces covered with an antimicrobial non-volatile agent, giving turbulence to the air flow, exposing it to ultraviolet light and returning the air to the enclosed space.

The disadvantage of this method is the need to use filtering devices. When a disinfection unit is of high productivity, its filters are either large-sized or (provided they are space-saving) with a major aerodynamic drag, which requires use of powerful air pumping devices: this also affects the weight and size of the unit and the amount of electrical power consumed. Besides, a large number of enclosed structures (in particular, vehicle interiors and elevator cabins) are characterized by air environment with a relatively high dust content. When used in sanitization devices, filters become contaminated very quickly and need to be replaced frequently.

There exists a well-known method for air sanitizaion (RU 2340360, cl A61L9/20, 2004) that comprises forming a flow of air, which presupposes filtration (from the air flow and through a HEPA filter) and removal of solid particles and microorganisms that exceed the preselected filter pore diameter, prior to the treatment of the specified air flow with UV rays, followed by the treatment of the specified air flow in the chamber with the UV rays emitted by at least one first source of UV radiation, aiming to eliminate the microorganisms present in the air flow and regulating at least one of the following parameters of the object: air flow rate; air humidity in the UV light treatment chamber; power output of the first source of UV radiation, with the aim to protect the above-mentioned first source of UV radiation from overheating or insufficient cooling; temperature of the first source of UV radiation, with the aim to protect the source of UV radiation from overheating or insufficient cooling; temperature of the air leaving the UV light treatment chamber (the specified temperature serves as a criterion for measuring the amount of UV radiation emitted by the microorganisms); temperature in the UV light treatment chamber (control is performed to ensure the specified temperature is maintained at a pre-set level) and the air sanitization device in accordance with the number of microorganisms determined by the microbial sensor.

Control is carried out to ensure that the microorganisms receive, at least, a fixed minimum dose of UV radiation that leads to their elimination.

The known method can be applied in enclosed structures, such as rooms in residential buildings or apartment blocks, - or any other premises where people or animals dwell and numerous contaminants (dust and various microorganisms, e.g. viruses, bacteria and fungi) are present. These pollutants pose risks to the health of humans and animals living in such enclosed structures. However, this method cannot be used to sanitize the air environment in elevators, due to the device being, in terms of its size, out of proportion to the elevator cabin. Moreover, this equipment requires frequent replacement of its components (filters included), which does not correspond to the frequency of routine maintenance of elevators and replacable components of recirculators, where the replacement period must constitute at least 8000 hours. KR20060018556 A and RU194640 U1 disclose sanitization methods and devices according to prior art.

The problem of the invention is the improvement of the known method with the possibility of its use for elevator cabin sanitization.

The technical result of the invention is the higher efficiency of elevator cabin sanitization performed within a short period of time.

The solution of the problem and the planned technical result are achieved by a method for sanitizing air in an elevator cabin by UV radiation according to claim 1.

In this case, the air flow recirculation ratio (Rr) is chosen to be the ratio of the recirculator capacity (in m³/h) to the elevator cabin capacity (in m³).

When the germicidal lamp power is less than 0.03 W per 1 m³, the accelerated reduction of elevator cabin air contamination to the required level cannot be ensured. When the power exceeds 0.12 W per 1 m³, the weight and size of air sanitization device (as well as the electrical power consumed) increase to a great extent; however, there is no significant increase in the speed of sanitization.

Therefore, lower ratio of air flow recirculation and its sanitization time does not ensure the necessary level of rapid reduction of air contamination in an elevator cabin. When this ratio is bigger, the weight and size characteristics of the device and its energy consumption become unreasonably increased, while no significant acceleration of sanitization is achieved.

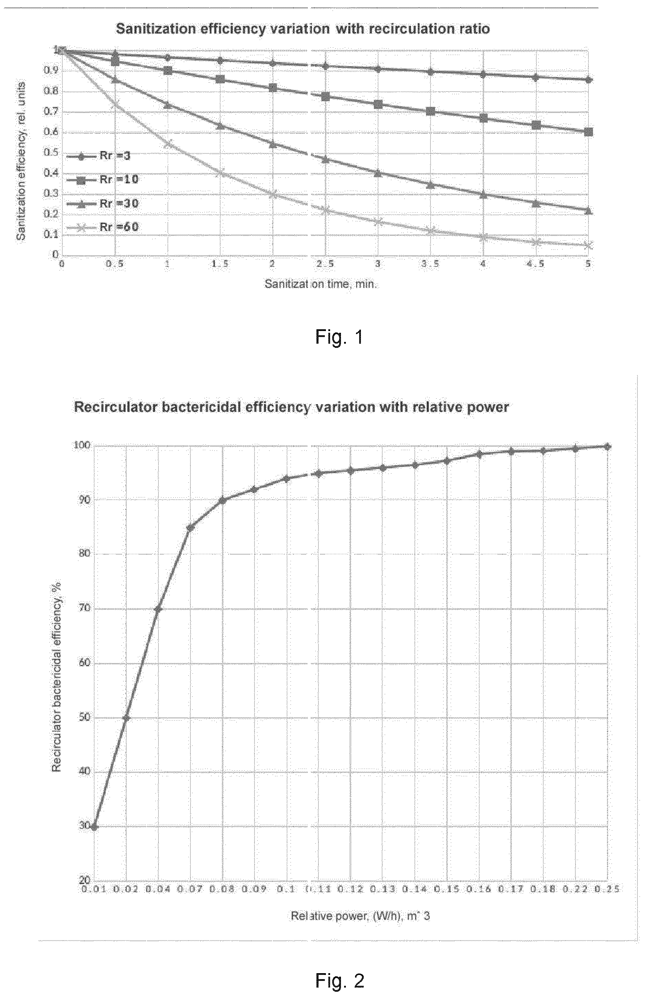

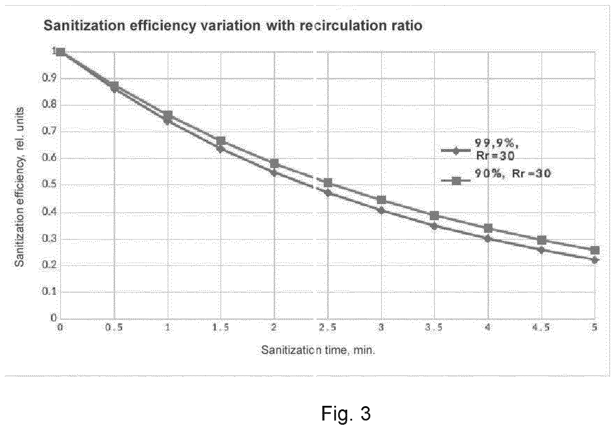

The method for sanitizing air in an elevator cabin is illustrated by the following graphs, where Fig. 1 explains the air environment sanitization efficiency variation (the ratio of surviving microorganisms to their original amount) within 5 minutes of work performed by recirculation units with various recirculation ratios; Fig. 2 illustrates the variation of the efficiency of sanitizing the air that passes through the recirculator sanitization chamber, with the relative power (the ratio of the recirculator UV source (W) power to the volume of air pumped per hour (m³); Fig. 3 shows the air environment sanitization efficiency variation (the ratio of surviving microorganisms to their original amount) within 5 minutes of work performed by recirculation units of the same capacity that provides for the recirculation ratio of 30 h⁻¹, but with a different sanitization efficiency of air per single passage through the recirculator.

The graph (Fig. 1) demonstrates that the use of traditional recirculators (with the recirculation ratio not exceeding 3 h⁻¹) almost does not lead to the reduction in the number of microorganisms in the air within 5 minutes of such device operation. Even when using devices with the recirculation ratio of 10 h⁻¹, only a 40% reduction in the number of microorganisms occurs within 5 minutes of the device operation.

Only when the recirculation ratio exceeds 30 h⁻¹, a noticeable (80% or more) reduction in the number of microorganisms is achieved. Thus, the performance of the recirculator used for elevator cabin air sanitization must ensure the recirculation ratio of 30 - 60 h⁻¹, specifically for the claimed invention of 40 h⁻¹.

Fig. 2 illustrates, in the form of a graph, the variation of the efficiency of sanitizing the air that passes through the recirculator sanitization chamber, with the relative power (the ratio of the recirculator UV source (W) power to the volume of air pumped per hour (m³). The graph shows that increasing the reduced power over 0.12 W/m³ per hour hardly leads to any increase in the efficiency of sanitization, while decreasing the reduced power to values below 0.3 W/m³ per hour causes a significant decrease in the efficiency of sanitization. With high recirculation ratios, changes in the efficiency of air sanitization per single passage through the recirculator have a weak effect on the concentration of microorganisms in the air.

Fig. 3 illustrates, in the form of a graph, the air sanitization efficiency variation. One of the two instances illustrates the use of a traditional recirculator, its bactericidal radiation sources emiting UV radiation of 0.24 W per 1 m³ per hour, of the air pumped through the recirculator, which ensures 99.9% sanitization efficiency per single passage. The other instance illustrates the use of another kind of recirculator, its bactericidal radiation sources emiting UV radiation of 0.08 W per 1 m³ per hour, of the air pumped through the recirculator (i.e. 3 times less than in the traditional one), which ensures 90% sanitization efficiency per single passage.

The graphs demonstrate that when comparing a 5-minute-long operation of both devices in an enclosed structure, it becomes obvious that their efficiency indices are, in terms of sanitization, almost identical.

Thus, the power of the UV radiation of the bactericidal radiation sources used in the device must be within 0.03 - 0.12 W per 1 m³/hour, specifically 0,08 or 0,12 according to the invention, of the air pumped through the air recirculator.

The method for sanitizing the air of an elevator cabin is illustrated by the following examples.

### Example 1.

Elevator cabin with a load capacity of 400 kg.

Cabin dimensions: 1100 mm x 1000 mm x 2100 mm.

Elevator cabin cubic space: 2.31 m³.

The capacity of the air pumping device is 90 m³/h, which ensures the recirculation ratio of 40 h⁻¹

The power of the bactericidal radiation source is 7 W (0.08 W per 1 m³/hour, of the air pumped through the air recirculator).

As a result of air sanitization procedures, after 4 minutes of operation of the device, the bactericidal efficiency was 80% (which means 5-time reduction in the contamination of the elevator cabin air).

These sanitization modes meet the requirements for the operation of elevator cabins.

### Example 2, not according to the invention.

The dimensions of the elevator cabin are the same as in Example 1, but the air pumping capacity of the recirculator corresponds to 30 m³/h, while the recirculation ratio is 13 h⁻¹. The power of the bactericidal radiation source is 3 W (0.1 W per 1 m³/hour, of the air pumped through the air recirculator).

As a result of air sanitization procedures, the bactericidal efficiency was 80% (which means 5-time reduction in the contamination of the elevator cabin air) only upon 15 minutes of the device operation, which does not meet the operating requirements due to the longer-than-required sanitization process.

### Example 3, not according to the invention.

The dimensions of the elevator cabin are the same as in Example 1, but the capacity of the air pumping recirculator corresponds to 150 m³/h, while the recirculation ratio is 65 h⁻¹. The power of the bactericidal radiation source is 15 W (0.1 W per 1 m³/hour, of the air pumped through the air recirculator).

The efficiency of air sanitization was 80% (which means 5-time reduction in the contamination of the elevator cabin air) upon 3.5 minutes of the device operation, which almost coincides with the results obtained in Example 1.

Thus, exceeded performance of air pumping devices significantly increases the cost of sanitization process due to the unreasonable power consumption by the equipment.

### Example 4.

Elevator cabin with a load capacity of 1600 kg.

Cabin dimensions: 1400 mm x 2400 mm x 2300 mm.

Elevator cabin cubic space: 7.7 m³.

The capacity of the air pumping device is 300 m³/h, which ensures the recirculation ratio of 40 h⁻¹.

The power of the bactericidal radiation source is 25 W, i.e. 0.08 W per 1 m³/hour, of the air pumped through the air recirculator.

As a result of cabin air sanitization, the bactericidal efficiency reached the level of 80% (which means 5-time reduction in the contamination of the elevator cabin air) within 4 minutes of the device operation. The sanitization modes shown in the example meet the operating requirements.

### Example 5, not according to the invention.

The dimensions of the elevator cabin are the same as in Example 4, but the air pumping capacity of the recirculator was 300 m³/h, which ensured the recirculation ratio of 40 h⁻¹.

The power of the bactericidal radiation source is 10 W (0.03 W per 1 m³/hour, of the air pumped through the air recirculator).

As a result of cabin air sanitization procedures, the bactericidal efficiency was 80% (which means 5-time reduction in the contamination of the elevator cabin air) only upon 15 minutes of the device operation.

Therefore, the sanitization performed under these modes turns out to be is ineffective due to the long period of time needed to get the required effect. This sanitization mode is not acceptable for elevator cabins. Example 6, not according to the invention.

The dimensions of the elevator cabin are the same as in Example 4, but the air pumping capacity of the recirculator was 300 m³/h, which ensured the recirculation ratio of 40 h⁻¹.

The power of the bactericidal radiation source is 75 W (0.25 W per 1 m³/hour, of the air pumped through the air recirculator).

As a result of air sanitization procedures, the bactericidal efficiency comprised 80% (which means 5-time reduction in the contamination of the elevator cabin air) within 3.5 minutes of the device operation, which almost coincides with the results obtained in Example 4.

### Example 7.

Elevator cabin with a load capacity of 1600 kg.

Cabin dimensions: 1400 mm x 2400 mm x 2300 mm.

Elevator cabin cubic space: 7.7 m³.

The capacity of the air pumping device is 300 m³/h, which ensures the recirculation ratio of 40 h⁻¹.

The power of the bactericidal radiation source is 36 W, i.e. 0.12 W per 1 m³/hour, of the air pumped through the air recirculator.

As a result of cabin air sanitization, the bactericidal efficiency reached the level of 80% (which means 5-time reduction in the contamination of the elevator cabin air) within 4 minutes of the device operation. The sanitization modes shown in the example meet the operating requirements.

Thus, the exceeded performance of air pumping devices significantly increases the cost of sanitization process due to the unreasonable power consumption by the equipment.

Up to the present moment, the method for sanitizing air in elevator cabins has passed industrial tests and has shown high efficiency of air sanitization in enclosed structures (characterized by a high level of air contamination) performed within a short period of time - from 3 to 5 minutes, specifically 4 minutes according the invention.

## Claims

1. - Method for sanitizing air in an elevator cabin by UV radiation that presupposes forming a flow out of the air present in an enclosed structure of an elevator cabin using a recirculator, and further pumping of the air flow through a chamber with a germicidal lamp in order to undergo sanitization with ultraviolet radiation, **characterized in that** the air flow recirculation ratio defined as the ratio of the recirculator capacity (in m³/h) to the elevator cabin capacity (in m³) is 40 h⁻¹, the lamp power being 0.08 or 0.12 W per 1 m³ of the air flow pumped per hour and the sanitization time is 4 minutes.

## Patentansprüche

1. - Verfahren zum Desinfizieren von Luft in einer Aufzugskabine durch UV-Strahlung, das ein Bilden eines Ausströmens der Luft, die in einer geschlossenen Struktur einer Aufzugskabine vorhanden ist, unter Verwendung eines Rezirkulators und ferner Pumpen des Luftstroms durch eine Kammer mit einer keimabtötenden Lampe voraussetzt, um einer Desinfektion mit ultravioletter Strahlung unterzogen zu werden, **dadurch gekennzeichnet, dass** das Luftstromzirkulationsverhältnis, definiert als das Verhältnis zwischen der Zirkulationskapazität (in m³/h) und der Aufzugskabinenkapazität (in m³), 40 h⁻¹ ist, wobei die Lampenleistung 0,08 oder 0,12 W pro 1 m³ des gepumpten Luftstroms pro Stunde ist und die Desinfektionszeit 4 Minuten ist.

## Revendications

1. - Procédé d'assainissement de l'air dans une cabine d'ascenseur par rayonnement UV qui présuppose la formation d'un flux à partir de l'air présent dans une structure fermée d'une cabine d'ascenseur à l'aide d'un recirculateur, puis le pompage du flux d'air à travers une chambre avec une lampe germicide afin de subir un assainissement par rayonnement ultraviolet, **caractérisé par le fait que** le rapport de recirculation du flux d'air défini comme le rapport de la capacité du recirculateur (en m³/h) à la capacité de la cabine d'ascenseur (en m³) est de 40h⁻¹, la puissance de la lampe étant de 0.08 ou 0,12 W par 1 m³ de flux d'air pompé par heure, et la durée d'assainissement étant de 4 minutes.
